Europäisches Patentamt

European Patent Office

Office européen des brevets

(11) Veröffentlichungsnummer: **0 203 276**
**A1**

(12) EUROPÄISCHE PATENTANMELDUNG

(21) Anmeldenummer: 86102776.1

(22) Anmeldetag: 03.03.86

(51) Int. Cl.⁴: **C07C 45/46** , C07C 49/788 ,
C07C 49/76

(30) Priorität: 25.05.85 DE 3519009

(43) Veröffentlichungstag der Anmeldung:
03.12.86 Patentblatt 86/49

(84) Benannte Vertragsstaaten:
DE FR GB IT NL

(71) Anmelder: **Rütgerswerke Aktiengesellschaft**
**Mainzer Landstrasse 217**
**D-6000 Frankfurt a.Main 1(DE)**

(72) Erfinder: **Steinbach, Rainer, Dr.**
**Bochumer Strasse 65**
**D-4620 Castrop-Rauxel(DE)**
Erfinder: **Ruppert, Ingo, Dr.**
**Nordstrasse 7**
**D-5300 Bonn(DE)**
Erfinder: **Schlich, Klaus**
**Falterer Pfad 13**
**D-5480 Remagen(DE)**

(54) **Verfahren zur selektiven Acylierung aromatischer Verbindungen.**

(57) Alkyl-oder arylsubstituierte Aromaten werden acyliert durch Umsetzung mit Carbonsäuren oder Carbonsäureanhydriden, -estern oder -halogeniden bei Temperaturen im Bereich von -50 bis +50 °C in kondensiertem Fluorwasserstoff.

EP 0 203 276 A1

## Verfahren zur selektiven Acylierung aromatischer Verbindungen

Die Erfindung betrifft ein neues Verfahren zur selektiven Acylierung von aromatischen Verbindungen, die mit Alkyl-oder Arylgruppen substituiert sind.

Es ist bekannt, alkylsubstituierte Aromaten durch Umsetzung mit Carbonsäuren, Carbonsäureanhydriden, -estern oder -halogeniden zu acylieren, wobei als Katalysatoren Friedel-Crafts-Katalysatoren verwendet werden.

Mittels Friedel-Crafts-Katalysatoren durchgeführte Reaktionen sind dafür bekannt, daß neben dem gewünschten Hauptprodukt relativ viele Nebenprodukte entstehen, was häufig zu gänzlich unerwünschten Harzbildungen führt. Friedel-Crafts-Reaktionen sind demnach wenig selektiv. Noch ausgeprägter ist dieser Nachteil, wenn man anstelle einkerniger aromatischer Verbindungen mehrkernige Aromaten acylieren will, da hier die Substitutionsmöglichkeiten noch größer sind.

Da derartige acylierte, aromatische Verbindungen aber oft als Zwischenprodukte für pharmazeutische Produkte, Insektizide, Farben oder Kunststoffe dienen, wobei eine hohe Reinheit gefordert wird, werden Acylierungsverfahren benötigt, die insbesondere bei mehrkernigen Aromaten zu hohen Ausbeuten möglichst reiner acylierter Produkte führen.

Aufgabe der Erindung ist daher ein Verfahren zur selektiven Acylierung aromatischer Verbindungen, die mit Alkyl-bzw. Arylgruppen substituiert sind, bei dem in hoher Ausbeute und Selektivität einkernige Alkyl-Aromaten in para-Stellung, zweikernige, in 2-Stellung substituierte Alkyl-Aromaten in 6-Stellung und dreikernige, in 2-Stellung substituierte Alkyl-Aromaten in 7-Stellung acyliert werden, wobei es zusätzlich notwendig ist, daß sich die weiteren entstehenden Reaktionsprodukte leicht vom Hauptprodukt abtrennen lassen.

Die Aufgabe wird gelöst durch ein Verfahren gemäß der Ansprüche 1 bis 5.

Es wurde gefunden, daß alkyl-oder arylsubstituierte Aromaten nahezu quantitativ mit Carbonsäuren, Carbonsäureanhydriden, -estern oder -halogeniden acyliert werden können, wobei in hoher Ausbeute und Selektivität bei entsprechenden einkernigen Aromaten in 4-Stellung, bei in 2-Stellung substituierten zweikernigen Aromaten in 6-Stellung und bei in 2-Stellung substituierten dreikernigen Aromaten in 7-Stellung eine Acylierung erfolgt, wenn diese Aromaten in kondensiertem Fluorwasserstoff gelöst und bei Temperaturen im Bereich von -50 bis +50 °C mit Carbonsäure oder einem Carbonsäureanhydrid, -ester oder -halogenid ohne Verwendung eines weiteren Katalysators umgesetzt werden.

Dabei entstehende Nebenprodukte können bei der Aufarbeitung des Reaktionsgemisches, die in an sich bekannter Weise erfolgt, leicht abgetrennt werden, so daß acylierte Produkte hoher Isomerenreinheit erhalten werden. So wird z.B. Toluol zu Acyl-4-methylbenzol, Diphenyl zu Acyl-4-phenyl-benzol, 2-Methyl-naphthalin zu 2-Acyl-6-methyl-naphthalin oder 2-Methyl-anthracen zu 2-Acyl-7-methylanthracen acyliert.

Als einkernige aromatische Verbindungen können erfindungsgemäß alle alkyl-, cycloalkyl-, aryl-oder aralkylsubstituierten Benzole erfindungsgemäß acyliert werden. Zwei-und dreikernige Aromaten, die erfindungsgemäß eingesetzt werden können, sind Naphthaline, Anthracene oder Phenanthrene, die in 2-Stellung alkylsubstituiert sind. Obwohl auch mit langen Alkylketten substituierte Aromaten erfindungsgemäß acyliert werden können, haben die bevorzugten Alkylsubstituenten 1 bis 4 C-Atome.

Lösemittel und offensichtlich auch Katalysator ist kondensierter, wasserfreier Fluorwasserstoff, der in etwa 5 bis 200-fachem molarem Überschuß eingesetzt wird.

Nach beendeter Umsetzung kann der Fluorwasserstoff entweder destillativ abgetrennt oder durch Umsetzung mit einer Lauge neutralisiert werden.

Acylierungsmittel können sowohl die freien Carbonsäuren sein als auch Carbonsäurederivate wie -anhydride, -ester oder -halogenide. Die bevorzugten Carbonsäuren bzw. -derivate sind solche mit 2 bis 4 C-Atomen.

Die Umsetzung wird in einer gegen Fluorwasserstoff beständigen Apparatur durchgeführt, bei der je nach gewünschter Reaktionsweise bei Normaldruck oder mit einem Überdruck gearbeitet werden kann.

Zur Umsetzung wird die zu acylierende aromatische Verbindung in kondensiertem Fluorwasserstoff gelöst. Danach wird unter Rühren die zur aromatischen Verbindung etwa äquimolare Menge an Acylierungsmittel zugegeben, wobei die Reaktionstemperatur im Bereich von -50 bis +50 °C, bevorzugt bei -10 bis +10 °C gehalten wird. Bei Reaktionstemperaturen bis etwa +10 °C kann unter Normaldruck gearbeitet werden. Bei höheren Temperaturen muß bei einem Druck bis etwa 5 bar umgesetzt werden. Nach beendeter Reaktion, was je nach gewählter Reaktionstemperatur nach 1 bis 10 h erreicht ist, wird das Reaktionsgemisch in an sich bekannter Weise entweder destillativ oder durch Extraktion aufgearbeitet.

## Beispiel 1

142 g (1 Mol) 2-Methylnaphthalin werden in 2 l kondensiertem HF gelöst. Anschließend werden 76 g (1,2 Mol) Acetylfluorid bei 0 °C zugegeben und unter Rühren die Temperatur 4 h gehalten. Das Gemisch wird vorsichtig auf ein Calciumhydroxid-Eis-Gemisch gegossen, die organische Phase abgenommen und destillativ aufgearbeitet.

Ausbeuten an 2-Acetyl-6-methylnaphthalin: 80 % bezogen auf eingesetztes Methylnaphthalin.

## Beispiel 2

Analog Beispiel 1 wird 2-Methylnaphthalin acyliert, wobei als Acylierungsmittel Acetanhydrid verwendet wird.

Ausbeute an 2-Acetyl-6-methylnaphthalin: 84 % bezogen auf eingesetztes Methylnaphthalin.

## Beispiel 3

Analog Beispiel 1 wird 2-Isopropylnaphthalin acyliert. Ausbeute an 2-Acetyl-6-isopropylnaphthalin: 85 % bezogen auf eingesetztes Isopropylnaphthalin.

## Ansprüche

1. Verfahren zur selektiven Acylierung aromatischer Verbindungen, die mit Alkyl-oder Arylgruppen substituiert sind, mit Carbonsäuren oder Carbonsäureanhydriden, -estern oder -halogeniden als Acylierungsmittel, **dadurch gekennzeichnet,** daß die aromatischen Verbindungen in kondensiertem Fluorwasserstoff gelöst und bei Temperaturen im Bereich von -50 bis +50 °C mit dem Acylierungsmittel umgesetzt werden.

2. Verfahren nach Anspruch 1,**dadurch gekennzeichnet**, daß die Umsetzung bei Temperaturen im Bereich von -10 bis +10 °C durchgeführt wird.

3. Verfahren nach Anspruch 1 und 2, **dadurch gekennzeichnet,** daß die Umsetzung im Druckbereich von Normaldruck bis 5 bar durchgeführt wird.

4. Verfahren nach Anspruch 1 bis 3, **dadurch gekennzeichnet,** daß mehrkernige in 2-Stellung alkylsubstituierte aromatische Verbindungen acyliert werden.

5. Verfahren nach Anspruch 1 bis 3, **dadurch gekennzeichnet,** daß 2-Alkylnaphthaline acyliert werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| Y | JOURNAL OF THE AMERICAN CHEMICAL SOCIETY, Band 61, Juli 1939, Seiten 1795,1796, State College, Pennsylvania, US; J.H. SIMONS et al.: "Hydrogen Fluoride as a Condensing Agent. VII. The Acylation of Aromatic Compounds" * Seiten 1795,1796 * | 1 | C 07 C 45/46<br>C 07 C 49/788<br>C 07 C 49/76 |
| | --- | | |
| Y | US-A-3 234 286 (F.R. LAWRENCE) * Patentansprüche * | 1-5 | |
| | --- | | |
| A | FR-A-2 238 696 (HOECHST AG) * Patentansprüche * | 1 | |
| | --- | | |
| A | FR-A-1 547 220 (KOPPERS CO INC.) * Patentansprüche * | 1 | |
| | --- | | RECHERCHIERTE SACHGEBIETE (Int. Cl.4) |
| A | DE-A-2 806 563 (HOECHST AG) * Patentansprüche * | 1 | C 07 C 45/00<br>C 07 C 49/00 |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 20-06-1986 | BONNEVALLE E.I.H. |